# EUROPEAN PATENT APPLICATION

(11) **EP 2 614 711 A1**
(43) Date of publication of application: **17.07.2013**
(21) Application number: 13163527.8
(22) Date of filing: 25.05.2004
(51) Int. Cl.: A01N 47/44, A61K 31/155, A61P 31/02

(54) **Disinfectant and/or bactericidal aqueous compositions**

(30) Priority: 28.05.2003 JP 2003151207; 30.06.2003 JP 2003188360
(62) Divisional of application: 11150449.4
(71) Applicant: OTSUKA PHARMACEUTICAL CO., LTD., Chiyoda-ku Tokyo 101-8535 (JP)
(72) Inventor: Nishibayashi, Toru, Tokushima, 771-0136 (JP); Sato, Tetsuya, Tokushima, 771-0205 (JP); Odomi, Masaaki, Tokushima, 771-1240 (JP); Ohguro, Kinue, Tokushima, 771-0142 (JP); Ishikawa, Hiroshi, Shiga, 520-0225 (JP)
(74) Representative: HOFFMANN EITLE

(57) **Abstract**

The present invention provides a disinfectant and/or bactericidal aqueous composition containing an olanexidine acid addition salt and a cyclodextrin.

## Description

### TECHNICAL FIELD

The present invention relates to disinfectant and/or bactericidal aqueous compositions containing an olanexidine acid addition salt.

### BACKGROUND ART

These days, people and articles frequently travel throughout the world, and hence there is an increased risk of pathogenic microorganisms that were previously restricted to a specific region or country being brought into all places throughout the world. Moreover, in modern life, people become increasingly hygiene-oriented and a hygienic environment has come to be provided. On the contrary, however, it is said that people's immune function is dropping.

Under the circumstances, to protect people and animals from pathogenic microorganisms, there are demands for the development of disinfectants/bactericides that are safe and easy to use, and have stronger effects.

Olanexidine [1-(3,4-dichlorobenzyl)-5-octylbiguanide] and salts thereof are compounds having disinfectant/bactericidal action that have been developed to meet the above demands, and have a broader antibacterial spectrum than hitherto known monobiguanide derivatives (see Japanese Patent No. 2662343, claim 1, paragraphs 0018, 0019, 0067, etc.). In Japanese Patent No. 2662343, it is proposed that when a monobiguanide derivative or a salt thereof is used as a disinfectant, a prescribed amount thereof is dissolved, dispersed or suspended in a medium such as water or an organic solvent, thus obtaining a liquid preparation for external use, for example eye drops, nasal drops, a gargle, a cleansing agent or an abluent.

Olanexidine is a basic compound, and hence forms acid addition salts. However, for example, the solubility of olanexidine hydrochloride in water is extremely low and is 0.02 W/V% at 0°C, and at this concentration the bactericidal activity is prone to being influenced by various factors such as pH, serum, soap, temperature and so on. Therefore, there have been strong demands for an aqueous preparation that is not prone to being influenced by these factors and contains olanexidine or a salt thereof dissolved in high concentrations.

### DISCLOSURE OF THE INVENTION

As described above, olanexidine and salts thereof are useful as disinfectants and/or bactericides, but it is considered that the usefulness would be increased by further improving the following points.

That is, because disinfectants and/or bactericides are widely used as liquid preparations, if a preparation containing olanexidine or a salt thereof as a principal agent could be prepared in as high a concentration as possible, then this would be advantageous in terms of the bactericidal effect and convenience of use. Such a high-concentration preparation may be used as such in situations in which a strong bactericidal action is required, or may be used as a formulated solution to be diluted at the time of use, in which case the preparation volume can be reduced, which is advantageous from the standpoint of its distribution and storage.

A suitable dissolution aid is required to make the olanexidine concentration high, but for the preparation containing the dissolution aid, the high bactericidal action of the olanexidine must be maintained stably, the skin irritation must be suppressed as much as possible, and the preparation must be easy to use.

However, up to now, no high-concentration preparation of olanexidine or a salt thereof has been known for which the olanexidine is dissolved in the medium in high concentration, the bactericidal action is maintained high, and the skin irritability is low. It is thus an object of the present invention to provide such high-concentration preparations of olanexidine or a salt thereof.

To attain the above object, the present inventors carried out various researches on dissolution aids for dissolving an olanexidine acid addition salt, and as a result discovered that the above object can be attained by dissolving an olanexidine acid addition salt in a water-containing solvent in the presence of at least one polyoxyethylene-based nonionic surfactant selected from the group consisting of polyoxyethylene higher alkyl ethers and polyoxyethylene alkylphenyl ethers.

Based on these findings, the present inventors carried out further researches, and accomplished disinfectant and/or bactericidal aqueous compositions of the present invention (hereinafter referred to as "compositions of invention I").

That is, a composition of invention I is disinfectant and/or bactericidal aqueous composition containing an olanexidine acid addition salt, and at least one polyoxyethylene-based nonionic surfactant selected from the group consisting of polyoxyethylene higher alkyl ethers and polyoxyethylene alkylphenyl ethers.

Examples of the polyoxyethylene higher alkyl ethers are compounds represented by formula (1):

R₁-O-(CH₂CH₂O)ₘ-H (1)

wherein R₁ represents an alkyl group having 7 to 20 carbon atoms, and m represents an integer from 9 to 12.

Examples of the polyoxyethylene alkylphenyl ethers are compounds represented by formula (2): 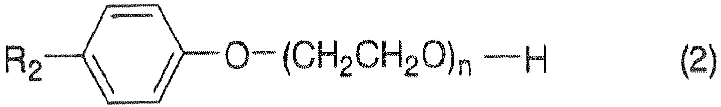 wherein R₂ represents an alkyl group having 7 to 20 carbon atoms, and n represents an integer from 9 to 12.

A preferable composition of invention I is an aqueous composition containing an olanexidine acid addition salt at a concentration of 0.05 to 5 W/V%, and a polyoxyethylene-based nonionic surfactant at a concentration of 0.1 to 10 W/V%.

The composition of invention I contains water as a solvent, and may further contain an alcohol insofar as it does not adversely affect the effect of the invention. When an alcohol is used, the concentration of the alcohol in the composition is 30 to 80 W/V%, preferably 60 to 80 W/V%. Examples of the alcohol include ethyl alcohol, isopropyl alcohol, denatured alcohol etc. Particularly ethyl alcohol is preferable.

Moreover, the present inventors also discovered that in a prescribed ethyl alcohol aqueous solution, a certain amount of olanexidine acid addition salt can be dissolved even in the absence of surfactants, and the disinfectant/bactericidal effect is improved.

Based on these findings, the present inventors carried out further researches, and accomplished disinfectant and/or bactericidal aqueous compositions of the present invention (hereinafter referred to as "compositions of invention II").

That is, a composition of invention II is an aqueous composition containing an olanexidine acid addition salt at a concentration of 0.05 to 0.5 W/V%, and an alcohol at a concentration of 20 to 80 W/V%, and not containing any surfactants. The concentration of the alcohol in the composition of invention II is preferably in the range of 30 to 60 W/V%. Examples of the alcohol include ethyl alcohol, isopropyl alcohol, denatured alcohol etc., and particularly ethyl alcohol is preferable.

Typically, the composition of invention II contains an olanexidine acid addition salt at a concentration of 0.05 to 0.5 W/V%, and an alcohol at a concentration of 20 to 80 W/V%, and not containing any surfactants.

Furthermore, the present inventors discovered that the above object can be attained by dissolving an olanexidine acid addition salt in water in the presence of at least one member selected from the group consisting of ester-based nonionic surfactants and cyclic oligosaccharides.

Based on these findings, the present inventors carried out further researches, and accomplished disinfectant and/or bactericidal aqueous compositions of the present invention (hereinafter referred to as "compositions of invention III").

That is, invention III relates to the following disinfectant and/or bactericidal aqueous compositions and the like.
1) A disinfectant and/or bactericidal aqueous composition containing an olanexidine acid addition salt, and at least one member selected from the group consisting of ester-based nonionic surfactants and cyclic oligosaccharides.
2) The disinfectant and/or bactericidal aqueous composition according to 1) above, wherein the ester-based nonionic surfactant is at least one member selected from the group consisting of (i) polyoxyethylene sorbitan fatty acid esters, (ii) polyglycerine fatty acid esters, (iii) polyoxyethylene glycerine fatty acid esters, and (iv) polyoxyethylene methyl glucoside fatty acid esters.
3) The disinfectant and/or bactericidal aqueous composition according to 2) above, wherein the polyoxyethylene sorbitan fatty acid esters are compounds represented by general formula (3): 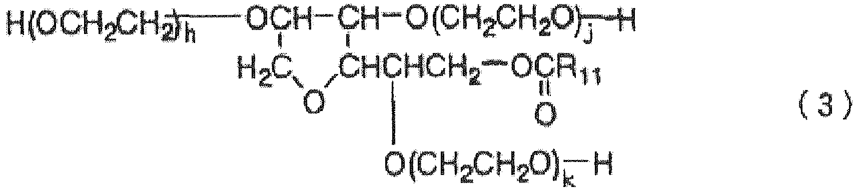 wherein R₁₁ represents an alkyl group having 10 to 20 carbon atoms, and each of h, j and k is an integer from 5 to 25.
4) The disinfectant and/or bactericidal aqueous composition according to 2) above, wherein the polyglycerine fatty acid esters are compounds represented by general formula (4): 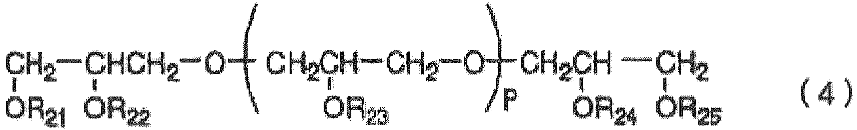 wherein one of R₂₁ to R₂₅ represents an alkanoyl group having 10 to 20 carbon atoms, and the other (p+3) represent a hydrogen atom, and p is an integer from 2 to 12.
5) The disinfectant and/or bactericidal aqueous composition according to 2) above, wherein the polyoxyethylene glycerine fatty acid esters are compounds represented by general formula (5): 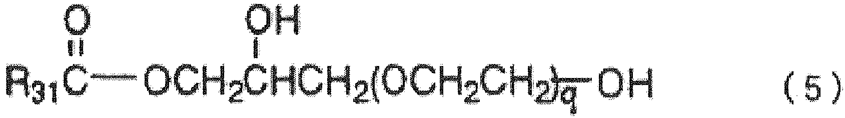 wherein R₃₁ represents an alkyl group having 6 to 16 carbon atoms, and q is an integer from 4 to 30.
6) The disinfectant and/or bactericidal aqueous composition according to 2) above, wherein the polyoxyethylene methyl glucoside fatty acid esters are compounds represented by general formula (6): 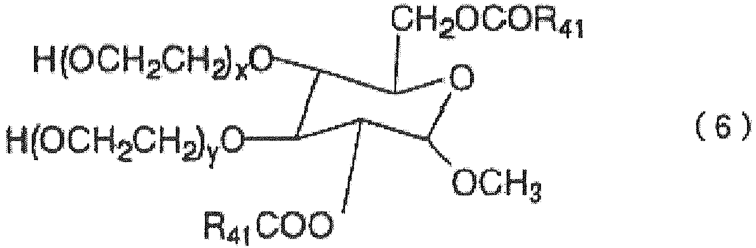 wherein R₄₁ represents an alkyl group having 15 to 20 carbon atoms, and sum of x and y is an integer from 20 to 160.
7) The disinfectant and/or bactericidal aqueous composition according to 1) above, wherein the cyclic oligosaccharide is a cyclodextrin.
8) The disinfectant and/or bactericidal aqueous composition according to 1) above, wherein the olanexidine acid addition salt is present at a concentration of 0.05 to 2.5 W/V% and the ester-based nonionic surfactant is present at a concentration of 0.1 to 10 W/V%.
9) The disinfectant and/or bactericidal aqueous composition according to 1) above, wherein the olanexidine acid addition salt is present at a concentration of 0.1 to 2.5 W/V% and the ester-based nonionic surfactant is present at a concentration of 0.1 to 10 W/V%.
10) The disinfectant and/or bactericidal aqueous composition according to 1) above, wherein the olanexidine acid addition salt is present at a concentration of 0.05 to 1 W/V% and the cyclic oligosaccharide is present at a concentration of 0.1 to 10 W/V%.
11) The disinfectant and/or bactericidal aqueous composition according to 1) above, wherein the olanexidine acid addition salt is present at a concentration of 0.1 to 1 W/V% and the cyclic oligosaccharide is present at a concentration of 0.1 to 10 W/V%.
12) The disinfectant and/or bactericidal aqueous composition according to 1) above further containing an alcohol.
13) The disinfectant and/or bactericidal aqueous composition according to 12) above, wherein the alcohol is present in the composition at a concentration of 20 to 80 W/V %.
14) A method of sterilizing or disinfecting an object, comprising contacting the object with an effective amount of the disinfectant and/or bactericidal aqueous composition according to 1) above.
15) Use of a disinfectant and/or bactericidal aqueous composition according to 1) above for sterilization or disinfection.

In the specification and claims, the concentration of each ingredient in a disinfectant and/or bactericidal aqueous composition is, unless otherwise indicated, expressed as a weight per volume percentage "% (W/V)", i.e., the weight (g) of each ingredient/100 mL of the disinfectant and/or bactericidal aqueous composition. The concentration "% (W/V)" is warranted at 0 °C, except in the case of special note.

In this specification and claims, "an aqueous composition" means a composition containing water. For example, the aqueous composition according to 8) above contains water as a solvent, an olanexidine acid addition salt at a concentration of 0.05 to 2.5 W/V% and the ester-based nonionic surfactant at a concentration of 0.1 to 10 W/V%.

### DETAILED DESCRIPTION OF THE INVENTION

The present invention will be described in detail.

### Olanexidine acid addition salt

"olanexidine acid addition salt" in the disinfectant and/or bactericidal aqueous composition of the present invention means a salt of olanexidine and an acid (organic acid or inorganic acid).

There are no particular restrictions on the acid forming the salt. Examples are organic acids such as formic acid, acetic acid, lactic acid, butyric acid, isobutyric acid, α-mercaptopropionic acid, trifluoroacetic acid, malic acid, fumaric acid, succinic acid, succinic monoamide, glutamic acid, tartaric acid, oxalic acid, citric acid, glycolic acid, gluconic acid, saccharic acid, ascorbic acid, penicillin, benzoic acid, phthalic acid, salicylic acid, anthranilic acid, benzenesulfonic acid, p-toluenesulfonic acid or methansulfonic acid, and inorganic acids such as hydrochloric acid, hydrobromic acid, sulfuric acid, phosphoric acid, nitric acid or carbonic acid. Moreover, there are no particular restrictions on the ratio between olanexidine and the acid forming the acid addition salt; in addition to 1:1, salts with various other ratios such as 1:2 can be used.

The acid addition salt is manufactured using an ordinary salt forming method such as directly mixing the acid and olanexidine together, dissolving one or both of the acid and olanexidine in a solvent such as water in advance and then mixing the solvent together, or putting the acid and olanexidine into a solvent such as water and carrying out dissolution and mixing.

Note that the acid addition salts of olanexidine indicated here can be used in any of the compositions of inventions I to III described below. In each composition of invention I to III, the olanexidine acid addition salt is dissolved in the solvent containing water and if desired, an alcohol. In this solution, the olanexidine and the acid may form a salt, or the two may exist in the free form, or the gluconic acid salt of olanexidine may coexist with free olanexidine and free gluconic acid.

### Compositions of invention I

A composition of invention I is a disinfectant and/or bactericidal aqueous composition containing an olanexidine acid addition salt, and at least one polyoxyethylene-based nonionic surfactant selected from the group consisting of polyoxyethylene higher alkyl ethers and polyoxyethylene alkylphenyl ethers.

The concentration of the olanexidine acid addition salt in the composition of invention I is preferably 0.05 to 5 W/V% in the composition. In this concentration range, the composition has a high disinfectant/bactericidal effect and a viscosity convenient to use. The concentration of the olanexidine acid addition salt is preferably in the range of 0.05 to 3.5 W/V%, more preferably 0.05 to 2 W/V%.

In the composition of invention I, the polyoxyethylene-based nonionic surfactant is selected from the group consisting of polyoxyethylene higher alkyl ethers and polyoxyethylene alkylphenyl ethers.

Preferable examples of the above-mentioned polyoxyethylene higher alkyl ethers are surfactants represented by previously mentioned formula (1), wherein the alkyl group represented by R₁ has 7 to 20 carbon atoms, preferably 9 to 14 carbon atoms, and include nonyl, decyl, undecyl, dodecyl, tridecyl and tetradecyl. Moreover, a mixture of a plurality of polyoxyethylene higher alkyl ethers having such alkyl groups may also be used. In the formula (1), m is a polymerization degree of oxyethylene unit (OE), and is an integer from 9 to 12.

Specific examples of such a surfactant include POE (9) lauryl ether (e.g., 'BL-9EX' made by Nikko Chemicals Co., Ltd.), POE (10) lauryl ether (e.g. 'Emulmin NL-100' made by Sanyo Chemical Industries, Ltd.) and POE (12) alkyl ether (e.g. 'Sannonic SS-120' made by Sanyo Chemical Industries, Ltd.). Here, the number in brackets after 'POE' indicates a polymerization degree of OE, i.e. m in formula (1).

Preferable examples of polyoxyethylene alkylphenyl ethers are surfactants represented by previously mentioned formula (2), wherein the alkyl group represented by R₂ has 7 to 20 carbon atoms, preferably 9 to 14 carbon atoms, with specific examples being the same group as the alkyl group represented by R₁ described above. In the formula (2), n is a polymerization degree of oxyethylene unit (OE), and is an integer from 9 to 12.

Specific examples of such surfactants are POE (10) nonylphenyl ether and POE (10) octylphenyl ether. Here, the number in brackets after 'POE' indicates a polymerization degree of OE, i.e. n in formula (2).

The composition of invention I contains such a polyoxyethylene-based nonionic surfactant in a solvent containing water, and therefore, it can be easily prepared as a liquid preparation having the olanexidine acid addition salt dissolved therein at a high concentration. Moreover the composition of invention I is such a preparation that the skin irritation can be suppressed and good feeling in use with good lubricity can be obtained.

The concentration of the polyoxyethylene-based nonionic surfactant in the composition of invention I is preferably 0.1 to 10 W/V%, particularly 0.15 to 6.5 W/V%, in the composition. Within such a range of the surfactant concentration, the disinfectant/bactericidal effect of the olanexidine acid addition salt can be stably maintained.

To make the composition of invention I into a liquid preparation, a solvent is required for dissolving the olanexidine acid addition salt in the presence of the polyoxyethylene-based nonionic surfactant. The solvent is preferably water, or a mixture of water and an alcohol (e.g., ethyl alcohol, isopropyl alcohol, denatured alcohol etc.). More preferable solvent is an aqueous solution of an alcohol, particularly an aqueous solution of ethyl alcohol. In this case, the concentration of ethyl alcohol present in the composition is advantageously in the range of 30 to 80 W/V%, preferably 60 to 80 W/V%. By making the ethyl alcohol concentration be within such a range, the liquid preparation containing the above-mentioned components can be made uniform, and can be used very conveniently because of its rapid disinfectant/bactericidal action at the time of use and its quick drying after use.

Thus, the composition of invention I is typically an aqueous composition. The aqueous composition is an aqueous solution of an olanexidine acid addition salt and a polyoxyethylene-based nonionic surfactant, the oranexidine acid addition salt being present at a concentration of 0.05 to 5 W/V% and the polyoxyethylene-based nonionic surfactant being present at a concentration of 0.1 to 10 W/V%.

Alternatively, the composition of invention I may be an alcohol-containing aqueous composition. Said alcohol-containing aqueous composition is a composition that is the same as the above aqueous composition except that a mixture of water and an alcohol (particularly ethyl alcohol) is used in place of water as the solvent. Typically, said alcohol-containing aqueous composition is an aqueous solution of an olanexidine acid addition salt, a polyoxyethylene-based nonionic surfactant and an alcohol (particularly ethyl alcohol), the oranexidine acid addition salt being present at a concentration of 0.05 to 5 W/V%, the polyoxyethylene-based nonionic surfactant being present at a concentration of 0.1 to 10 W/V% and the alcohol (particularly ethyl alcohol) being present at a concentration of 30 to 80 W/V%, preferably 60 to 80 W/V%.

In addition to the above components, if necessary, various other components may be included in the composition of invention I for the purpose of further improving the product quality.

For example, from the viewpoint of improving the skin protecting ability and moisturizing ability, one or more fatty acid triglycerides and, if necessary, hydroxypropylmethylcellulose are preferably included. By including such components, the effect of suppressing skin irritation is enhanced particularly in the case of using a solvent containing ethyl alcohol.

Regarding the above-mentioned one or more fatty acid triglycerides, any ones ordinarily used in the field of medicines/disinfectants can be used with no particular limitations thereto. In general, ones that are liquid at atmospheric pressure and normal temperatures around 20°C are preferable; examples are triglycerides of fatty acids such as isooctanoic acid, caprylic acid, capric acid, lauric acid, myristic acid, palmitic acid, palmitoleic acid, stearic acid, isostearic acid, oleic acid, linoleic acid and linolenic acid; one of these fatty acid triglycerides may be used alone, or two or more can be used in combination. Furthermore, a triglyceride having a vegetable oil such as olive oil or rapeseed oil as a raw material thereof may be used.

Out of the above-mentioned fatty acid triglycerides, especially isooctanoic acid triglyceride can be preferably selected because of its high safety and its better feeling in use.

Said one or more fatty acid triglycerides, when used, is preferably present at a concentration of 0.01 to 10 W/V%, particularly at a concentration in the range of 0.3 to 3 W/V%, in the composition. If the concentration is within such a range, then the moisturizing effect and the effect of suppressing skin irritation, skin roughness and so on will be exhibited sufficiently, and virtually no stickiness will remain on the surface of the skin.

In the case of using hydroxypropylmethylcellulose, it is preferable that the concentration thereof in the composition is in the range of 0.001 to 1.0 W/V%, particularly 0.005 to 0.5 W/V%.

Furthermore, one or more components conventionally added to disinfectants/bactericides can be added to the composition of invention I so long as the object thereof is not impaired. As described in Japanese Unexamined Patent Publication No. 11-199476, examples thereof are blood circulation promoters such as propylene carbonate, *N*-methyl-2-pyrrolidone (Japanese Pharmaceutical Excipients), propylene glycol, polysorbate 80 (Japanese Pharmacopoeia), and tocopherol acetate; tissue recovering agents such as allantoin; refreshing agents such as peppermint oil and 1-menthol; oily components such as liquid paraffin, methyl polysiloxane, methyl phenyl polysiloxane, and squalane; polyhydric alcohol such as glycerol, 1,3-butylene glycol, and sorbitol; pH regulators; etc.

The pH of the composition of invention I is preferably 4 to 8, more preferably 5 to 6, in the form of a formulated solution to be diluted at the time of use. If the pH is adjusted to such a range, then the composition will have good stability, and the feeling in use will be good.

The composition of invention I can be prepared, for example, by adding water to the olanexidine acid addition salt (e.g. olanexidine hydrochloride) and the polyoxyethylene-based nonionic surfactant(s) and carrying out mixing and dissolution, adjusting the mixture to a pH of approximately 4 to 8 using an acid such as dilute hydrochloric acid or the like. Such a composition can be used for disinfection or sterilization. If necessary, with such a composition being used as a formulated solution to be diluted at the time of use, a liquid preparation having a prescribed concentration can be prepared by diluting the composition with water or the like.

### Compositions of invention II

Next, a composition of invention II is an aqueous composition containing an olanexidine acid addition salt at a concentration of 0.05 to 0.5 W/V%, and an alcohol at a concentration of 20 to 80 W/V%, and not containing any surfactants in the composition. The alcohol concentration is preferably 30 to 60 W/V%. Examples of the alcohol include ethyl alcohol, isopropyl alcohol, denatured alcohol etc., and particularly ethyl alcohol is preferable.

Unlike with the composition of invention I, this composition does not contain a surfactant, but the disinfectant/bactericidal effect can be rapidly exhibited, and the rapidity of drying after use can be improved.

Typically, the composition of invention II is an aqueous composition containing an olanexidine acid addition salt at a concentration of 0.05 to 0.5 W/V% and an alcohol at a concentration of 20 to 80 W/V%, and not containing any surfactants.

In addition to the above components, if necessary, various other components may be included in the composition of invention II for the purpose of further improving the product quality.

For example, from the viewpoint of improving the skin protecting ability and moisturizing ability, one or more fatty acid triglycerides and, if necessary, hydroxypropylmethylcellulose are preferably included. By including such components, the effect of suppressing skin irritation is enhanced particularly in the case of using a solvent containing ethyl alcohol.

Regarding the above-mentioned one or more fatty acid triglycerides, any ones ordinarily used in the field of medicines/disinfectants can be used with no particular limitations thereto. In general, ones that are liquid at atmospheric pressure and normal temperatures around 20°C are preferable; examples are triglycerides of fatty acids such as isooctanoic acid, caprylic acid, capric acid, lauric acid, myristic acid, palmitic acid, palmitoleic acid, stearic acid, isostearic acid, oleic acid, linoleic acid and linolenic acid; one of these fatty acid triglycerides may be used alone, or two or more can be used in combination. Furthermore, a triglyceride having a vegetable oil such as olive oil or rapeseed oil as a raw material thereof may be used.

Out of the above-mentioned fatty acid triglycerides, especially isooctanoic acid triglyceride can be preferably selected because of its high safety and its better feeling in use.

Said one or more fatty acid triglycerides, when used, is preferably present at a concentration of 0.01 to 10 W/V%, particularly at a concentration in the range of 0.3 to 3 W/V%, in the composition. If the concentration is within such a range, then the moisturizing effect and the effect of suppressing skin irritation, skin roughness and so on will be exhibited sufficiently, and virtually no stickiness will remain on the surface of the skin.

In the case of using hydroxypropylmethylcellulose, it is preferable that the concentration thereof in the composition is in the range of 0.001 to 1.0 W/V%, particularly 0.005 to 0.5 W/V%.

Furthermore, one or more components conventionally added to disinfectants/bactericides can be added to the composition of invention II so long as the object thereof is not impaired. As described in Japanese Unexamined Patent Publication No. 11-199476, examples thereof are blood circulation promoters such as propylene carbonate, N-methyl-2-pyrrolidone (Japanese Pharmaceutical Excipients), propylene glycol, polysorbate 80 (Japanese Pharmacopoeia), and tocopherol acetate; tissue recovering agents such as allantoin; refreshing agents such as peppermint oil and 1-menthol; oily components such as liquid paraffin, methyl polysiloxane, methyl phenyl polysiloxane, and squalane; polyhydric alcohol such as glycerol, 1,3-butylene glycol, and sorbitol; pH regulators; etc.

The composition of invention II can be prepared, for example, by mixing the olanexidine acid addition salt (e.g., olanexidine hydrochloride) in an alcohol aqueous solution (e.g., 50 to 90% ethanol aqueous solution) and carrying out dissolution, and adjusting the solution to a pH of approximately 4 to 8 using an acid such as dilute hydrochloric acid or the like. Such a composition can be used for disinfection or sterilization. If necessary, with such a composition being used as a formulated solution to be diluted at the time of use, a liquid preparation having a prescribed concentration can be prepared by diluting the composition with water or the like.

### Compositions of invention III

A composition of invention III is a disinfectant and/or bactericidal aqueous composition containing an olanexidine acid addition salt, and at least one member selected from the group consisting of an ester-based nonionic surfactant and a cyclic oligosaccharide.

A composition of invention III is prepared by dissolving an olanexidine acid addition salt as a principal agent in a water-containing solvent in the presence of an ester-based nonionic surfactant and/or a cyclic oligosaccharide serving as a dissolution aid for the olanexidine acid addition salt.

The ester-based nonionic surfactant is a surfactant comprising non-electrolytic molecules which is an ester of a substance having a hydrophobic group (a fatty acid residue such as nonyl, decyl, undecyl, dodecyl, tetradecyl, etc.) and a substance having a hydrophilic group (a polyoxyethylene group, a hydroxyl group, etc.) that is nonionic and does not dissociate into ions, and is a surfactant having a property of increasing the solubility of the olanexidine acid addition salt in water.

In general, an ester-based nonionic surfactant having an HLB (hydrophile-lipophile balance) value of 8 to 20, preferably 12 to 16, can be used.

The composition of invention III preferably contains at least one member selected from the group consisting of an ester-based nonionic surfactant and a cyclic oligosaccharide at a concentration of 0.1 to 10 W/V% and an olanexidine acid addition salt at a concentration of 0.05 to 2.5 W/V%.

Examples of such ester-based nonionic surfactants include (i) polyoxyethylene sorbitan fatty acid esters, (ii) polyglycerine fatty acid esters, (iii) polyoxyethylene glycerine fatty acid esters, and (iv) polyoxyethylene methyl glucoside fatty acid esters.

As the above-mentioned polyoxyethylene sorbitan fatty acid esters, any of various types can be used, but of these, preferable are those represented by general formula (3): 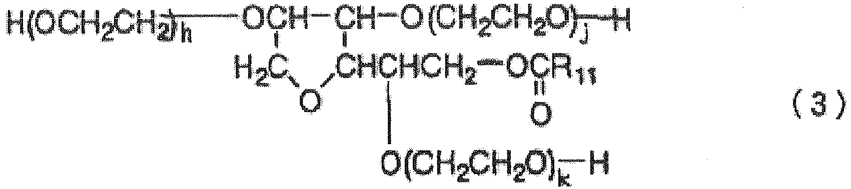 wherein R₁₁ represents an alkyl group having 10 to 20 carbon atoms, and each of h, j and k is an integer from 5 to 25.

R₁₁ is preferably a straight chain or branched chain alkyl group having 10 to 20 carbon atoms, with a straight chain or branched chain alkyl group having 10 to 14 carbon atoms being particularly preferable. Moreover, h, j and k may be the same or different, and each is preferably an integer from 5 to 25, particularly an integer from 5 to 20. Total number of h, j and k is an integer from 15 to 30, particularly 15 to 25.

Specific examples include a polyoxyethylene (POE) sorbitan monooleate, a polyoxyethylene (POE) sorbitan monostearate, a polyoxyethylene (POE) sorbitan monopalmitate and a polyoxyethylene (POE) sorbitan isostearate. Total number (h+j+k) of oxyethylene unit (OE) in each compound is preferably in the range of 15 to 30, more preferably 15 to 25, particularly 20.

In the composition of the present invention containing the polyoxyethylene sorbitan fatty acid ester, the content of the polyoxyethylene sorbitan fatty acid ester is preferably 0.1 to 10 W/V%, particularly 1.5 to 3 W/V%, and the content of the olanexidine acid addition salt is preferably 0.05 to 1.5 W/V%, particularly 0.5 to 1 W/V%, in the composition.

Examples of the alkyl group represented by R₁₁ include decyl group, undecyl group, dodecyl group, tridecyl group, tetradecyl group, pentadecyl group, heptadecyl group, octadecyl group, and nonadecyl group.

As the above-mentioned polyglycerine fatty acid esters, any of various types can be used, but of these, preferable are polyglycerine monofatty acid esters represented by general formula (4): 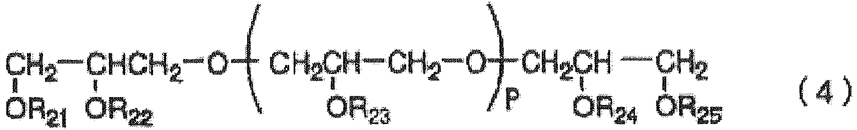 wherein one of R₂₁ to R₂₅ represents an alkanoyl group having 10 to 20 carbon atoms, and the other (p+3) represent a hydrogen atom, and p is an integer from 2 to 12. In other words, when one of R₂₁, R₂₂, R₂₄ and R₂₅ is an alkanoyl group, the other three are hydrogen and R₂₃s are hydrogen, and when one of (p) R₂₃s is an alkanoyl group, the other (p-1) R₂₃ s are hydrogen and R₂₁, R₂₂, R₂₄ and R₂₅ are hydrogen.

Said one of R₂₁ to R₂₅ is preferably a straight chain or branched chain alkanoyl group having 10 to 20 carbon atoms, with a straight chain or branched chain alkanoyl group having 5 to 16 carbon atoms being more preferable, and a straight chain or branched chain alkanoyl group having 10 to 14 carbon atoms being particularly preferable. Moreover, p is preferably an integer from 2 to 12, particularly preferably an integer from 8 to 10.

Specific examples include a polyglyceryl monolaurate such as decaglyceryl monolaurate; a polyglyceryl monooleate such as hexaglyceryl monooleate and decaglyceryl monooleate; and a polyglyceryl monostearate such as diglyceryl monostearate.

In the composition of the present invention containing the polyglycerine fatty acid ester, the content of the polyglycerine fatty acid ester is preferably 0.1 to 10 W/V%, particularly 3.0 to 6.0 W/V%, and the content of the olanexidine acid addition salt is preferably 0.05 to 1.5 W/V%, particularly 0.5 to 1 W/V%, in the composition.

Examples of the alkanoyl group represented by one of R₂₁ to R₂₅ include decanoyl group, undecanoyl group, dodecanoyl group, tridecanoyl group, tetradecanoyl group, pentadecanoyl group, heptadecanoyl group, octadecanoyl group, and nonadecanoyl group.

As the above-mentioned polyoxyethylene glycerine fatty acid esters, any of various types can be used, but of these, preferable are those represented by general formula (5): 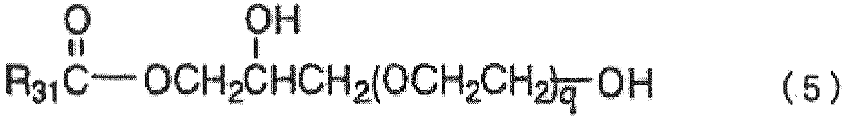 wherein R₃₁ represents an alkyl group having 6 to 16 carbon atoms, and q is an integer from 4 to 30.

R₃₁ is preferably a straight chain or branched chain alkyl group having 6 to 16 carbon atoms, with a straight chain or branched chain alkyl group having 8 to 10 carbon atoms being particularly preferable. q is preferably an integer from 4 to 30, particularly an integer from 6 to 15.

Specific examples include a polyoxyethylene glyceryl cocoate (q = 5 to 10, particularly 7), polyoxyethylene glyceryl caprylate/caprate (q =5 to 10, particularly 7)., polyoxyethylene glyceryl monolaurate (q = 5 to 10, particularly 7), and polyoxyethylene glyceryl monoisostearate (q = 10 to 20, particularly 15).

In the composition of the present invention containing the polyoxyethylene glycerine fatty acid ester, the content of the polyoxyethylene glycerine fatty acid ester is preferably 0.1 to 10 W/V%, particularly 2 to 5 W/V%, and the content of the olanexidine acid addition salt is preferably 0.05 to 2.5 W/V%, particularly 0.5 to 1 W/V%, in the composition

Examples of the alkyl group represented by R₃₁ include hexyl group, heptyl group, octyl group, nonyl group, decyl group, undecyl group, dodecyl group, tridecyl group, tetradecyl group, pentadecyl group, and hexadecyl group.

As the above-mentioned polyoxyethylene methyl glucoside fatty acid esters, any of various types can be used, but of these, preferable are polyoxyethylene methyl glucoside difatty acid esters represented by general formula (6): 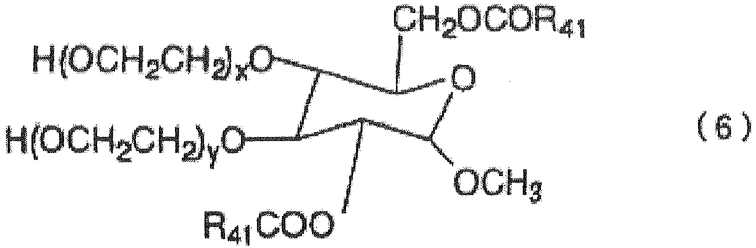 wherein R₄₁ represents an alkyl group having 15 to 20 carbon atoms, and sum of x and y is an integer from 20 to 160.

R₄₁ is preferably a straight chain or branched chain alkyl group having 15 to 20 carbon atoms, with a straight chain or branched chain alkyl group having 16 to 18 carbon atoms being particularly preferable. Moreover, x and y may be the same or different, and each is preferably an integer from 5 to 100, particularly an integer from 10 to 70. Total number of x and y is preferably an integer from 20 to 160, particularly an integer from 100 to 140.

Specific examples include a polyoxyethylene methyl glucoside dioleate, and a polyoxyethylene methyl glucoside distearate.

In the composition of the present invention containing the polyoxyethylene methyl glucoside fatty acid ester, the content of the polyoxyethylene methyl glucoside fatty acid ester is preferably 0.1 to 10 W/V%, particularly 5 to 10 W/V%, and the content of the olanexidine acid addition salt is preferably 0.05 to 1.5 W/V%, particularly 0.5 to 1 W/V%, relative to the composition

As the above-mentioned cyclic oligosaccharide, one that has a property of increasing the solubility of the olanexidine acid addition salt in water, and that does not impede the disinfectant/bactericidal action is selected. Out of these, cyclodextrins can be preferably used. Three kinds of cyclodextrins are known, namely α-cyclodextrin, β-cyclodextrin and γ-cyclodextrin, and in the present invention any of these can be used.

In the composition of the present invention containing the cyclic oligosaccharide, the content of the cyclic oligosaccharide (particularly cyclodextrin) is preferably 0.1 to 10 W/V%, particularly 1.5 to 4 W/V%, and the content of the olanexidine acid addition salt is preferably 0.05 to 1 W/V%, particularly 0.2 to 0.5 W/V%, relative to the composition.

Especially, when β-cyclodextrin is used, the content of the β-cyclodextrin is preferably 0.1 to 1.5 W/V% in the composition.

The ester-based nonionic surfactant or cyclic oligosaccharide is preferably used in a concentration range as described above; if such a concentration range is used, then a sufficient disinfectant/bactericidal effect will be exhibited.

As the solvent in the composition of invention III, water alone is preferable, but a solvent comprisiing water and an alcohol may be used. Examples of the alcohol include ethyl alcohol, isopropyl alcohol, denatured alcohol etc., and particularly ethyl alcohol is preferable. In this case, the concentration of the alcohol is set in 20 to 80 W/V%, particularly 30 to 60 W/V%, relative to the composition.

In addition to the above components, if necessary, various other components may be included in the composition of invention III for the purpose of further improving the product quality.

For example, from the viewpoint of improving the skin protecting ability and moisturizing ability, one or more fatty acid triglycerides and, if necessary, hydroxypropylmethylcellulose are preferably included. By including such components, the effect of suppressing skin irritation is enhanced particularly in the case of using a solvent containing ethyl alcohol.

Regarding the above-mentioned one or more fatty acid triglycerides, any ones ordinarily used in the field of medicines/disinfectants can be used with no particular limitations thereto. In general, ones that are liquid at atmospheric pressure and normal temperatures around 20°C are preferable; examples are triglycerides of fatty acids such as isooctanoic acid, caprylic acid, capric acid, lauric acid, myristic acid, palmitic acid, palmitoleic acid, stearic acid, isostearic acid, oleic acid, linoleic acid and linolenic acid; one of these fatty acid triglycerides may be used alone, or two or more can be used in combination. Furthermore, a triglyceride having a vegetable oil such as olive oil or rapeseed oil as a raw material thereof may be used.

Out of the above-mentioned fatty acid triglycerides, especially isooctanoic acid triglyceride can be preferably selected because of its high safety and its better feeling in use.

Said one or more fatty acid triglycerides, when used, is preferably present at a concentration of 0.01 to 10 W/V%, particularly at a concentration in the range of 0.3 to 3 W/V%, in the composition. If the concentration is within such a range, then the moisturizing effect and the effect of suppressing skin irritation, skin roughness and so on will be exhibited sufficiently, and virtually no stickiness will remain on the surface of the skin.

In the case of using hydroxypropylmethylcellulose, it is preferable that the concentration thereof in the composition is in the range of 0.001 to 1.0 W/V%, particularly 0.005 to 0.5 W/V%.

Furthermore, one or more components conventionally added to disinfectants/bactericides can be added to the composition of invention III so long as the object thereof is not impaired. As described in Japanese Unexamined Patent Publication No. 11-199476, examples thereof are blood circulation promoters such as propylene carbonate, *N*-methyl-2-pyrrolidone (Japanese Pharmaceutical Excipients), propylene glycol, polysorbate 80 (Japanese Pharmacopoeia), and tocopherol acetate; tissue recovering agents such as allantoin; refreshing agents such as peppermint oil and 1-menthol; oily components such as liquid paraffin, methyl polysiloxane, methyl phenyl polysiloxane, and squalane; polyhydric alcohol such as glycerol, 1,3-butylene glycol, and sorbitol; pH regulators; etc.

The pH of the composition of invention III is preferably 4 to 8, more preferably 5 to 6, in the form of a formulated solution to be diluted at the time of use. If the pH is adjusted to such a range, then the liquid preparation will have good stability, and moreover the feeling in use will be good.

The composition of invention III can be prepared, for example, by adding water to the olanexidine acid addition salt (e.g., olanexidine hydrochloride) and at least one member selected from the group consisting of the ester-based nonionic surfactant and cyclic oligosaccharide, carrying out mixing and dissolution, adjusting the mixture to a pH of approximately 4 to 8 using dilute hydrochloric acid or the like. Such a composition can be used for disinfection or sterilization. If necessary, with such a composition being used as a formulated solution to be diluted at the time of use, a liquid preparation having a prescribed concentration can be prepared by diluting the composition with a solvent such as water or an alcohol.

### Application of the compositions of inventions I to III

The disinfectant and/or bactericidal aqueous compositions of the present invention (the compositions of inventions I to III) described above have a broad antibacterial spectrum against various microorganisms. For example, the composition has an effective bactericidal and disinfectant action on gram-positive bacteria such as Staphylococci, Streptococci, Enterococci, and *Listeria* and *Propionibacterium* spp; and gram-negative bacteria such as *Escherichia coli, Shigella, Salmonella, Citrobacter, Klebsiella, Enterobacter, Serratia, Proteus, Morganella, Yersinia, Vibrio, Pseudomonas, Acinetobacter, Neisseria, Haemophilus,* and Bacteroides spp.

The composition also has an antiviral activity for virus with envelope such as Influenza virus, Human immunodeficiency virus, Herpes simplex virus, and Vesicular stomatitis virus, and has antifungal activity on yeast-like fungi such as *Candida* spp, *Cryptococcus neoformans,* and *Saccharomyces cerevisiae.*

The disinfectant and/or bactericidal aqueous composition of the present invention means an agent that can be widely used for the purpose of killing, decreasing, inhibiting etc. the various microbes such as described above.

The composition of the present invention exhibits bactericidal or disinfectant activity by contacting an object containing the microbes with an effective amount of the composition. Contacting methods are not particularly limited, and specific examples thereof include immersion, spraying, dry bath, etc. Examples of the object include skin and/or hands of human, animal skin, medical equipment, lavatories, bathrooms, furniture, articles and so on.

Therefore, the composition of the present invention can be suitably used for disinfecting skin/hands, preoperative skin, skin wounded, medical equipment, operating rooms, sickrooms, furniture, equipment and other articles, etc.

Moreover, the composition of the present invention may be used by impregnating it in a base fabric. Examples of such base fabrics include cotton wool, gauze, paper, non-woven cloths, towels, other cloths and so on. Usable such base fabrics may be water-decomposable or non-water-decomposable.

The composition of the present invention, which contains an olanexidine acid addition salt (olanexidine hydrochloride etc.) in a high concentration, can be used as a stock solution, and also can be used by diluting it with water or the like in accordance with the application.

### EFFECTS OF THE INVENTION

With a composition of invention I, the olanexidine acid addition salt as a principal agent can be dissolved in a solvent at a high concentration under the presence of at least one specific polyoxyethylene-based nonionic surfactant. Moreover, the composition of invention I maintains a high solubility of olanexidine acid addition salt with no reduction in the bactericidal activity thereof, has suppressed skin irritation, and exhibits a rapid bactericidal effect and a quick-drying property. Therefore, the composition can be widely used as disinfectant and/or bactericidal composition.

With an olanexidine acid addition salt alone, the solubility in a water is low, and the bactericidal activity is prone to being influenced by pH, organic matter, soap, temperature and so on. According to the composition of invention I, however, such influence can be eliminated, and hence the practical usefulness can be improved.

With a composition of invention II, the concentration of the olanexidine acid addition salt is not so high, but the disinfectant/bactericidal effect can be rapidly exhibited, and the rapidity of drying after use can be enhanced.

With a composition of invention III, the olanexidine acid addition salt as a principal agent can be dissolved in a solvent at a high concentration by using at least one member selected from the group consisting of an ester -based nonionic surfactant and a cyclic oligosaccharide. Compared with the composition using the olanexidine acid addition salt alone, the bactericidal activity of the composition of invention III is thus increased, and a bactericidal effect can be exhibited even in a short time.

With an olanexidine acid addition salt alone, the solubility in a water is low, and the bactericidal activity is prone to being influenced by pH, organic matter, soap, temperature and so on. According to the composition of invention III, however, such influence can be eliminated, and hence the practical usefulness can be improved. Moreover, with the composition of invention III, skin irritation is suppressed.

### BEST MODE FOR CARRYING OUT THE INVENTION

Preferable examples, preparation examples and test examples for the present invention will be described below; however, the present invention is not limited to these examples.

Note that in the examples, "%" means "% (W/V)" unless otherwise stated. The preparation of a bactericidal and/or disinfectant aqueous composition in each example was carried out at room temperature (Approximate at 25°C).

### Compositions of invention I and invention II

### Example 1

0.5 g of an olanexidine hydrochloride powder (Otsuka Pharmaceutical Co., Ltd.) was mixed and dissolved into 74 ml of 95% ethyl alcohol (Wako Pure Chemical Industries, Ltd.), the pH was adjusted to 6 using dilute hydrochloric acid, and then water was added to give a total volume of 100 mL, thus obtaining a disinfectant and/or bactericidal aqueous composition.

### Example 2

Approximately 85 ml of water was added to 0.1 g of an olanexidine hydrochloride powder (Otsuka Pharmaceutical Co., Ltd.) and 0.32 g of POE (10) nonylphenyl ether (made by Nikko Chemicals Co., Ltd.) and mixing and dissolution were carried out, the pH was adjusted to 5, and then water was further added to give a total volume of 100 mL, thus obtaining a disinfectant and/or bactericidal aqueous composition.

### Example 3

Approximately 85 ml of water was added to 0.1 g of an olanexidine hydrochloride powder (Otsuka Pharmaceutical Co., Ltd.) and 0.32 g of POE (9) lauryl ether (made by Nikko Chemicals Co., Ltd.) and mixing and dissolution were carried out, the pH was adjusted to 5, and then water was further added to give a total volume of 100 mL, thus obtaining a disinfectant and/or bactericidal aqueous composition.

### Example 4

Approximately 85 ml of water was added to 0.1 g of an olanexidine hydrochloride powder (Otsuka Pharmaceutical Co., Ltd.) and 0.32 g of POE (10) lauryl ether (made by Sanyo Chemical Industries, Ltd.) and mixing and dissolution were carried out, the pH was adjusted to 5, and then water was further added to give a total volume of 100 mL, thus obtaining a disinfectant and/or bactericidal aqueous composition.

### Example 5

Approximately 85 ml of water was added to 0.1 g of an olanexidine hydrochloride powder (Otsuka Pharmaceutical Co., Ltd.) and 0.41 g of a POE (12) alkyl (C₁₂-C₁₄) ether (made by Sanyo Chemical Industries, Ltd.) and mixing and dissolution were carried out, the pH was adjusted to 6, and then water was further added to give a total volume of 100 mL, thus obtaining a disinfectant and/or bactericidal aqueous composition.

The aqueous solutions obtained in Examples 1 to 5 were still transparent and colorless without precipitation even after being left at a temperature in the vicinity of 0°C

### Control Example 1

74 ml of ethanol for disinfection was added to 2.5 ml of a 20 W/V% chlorhexidine gluconate aqueous solution (Sumitomo Pharmaceuticals Company Limited) and mixing and dissolution were carried out sufficiently, the pH was adjusted to 6.0 using gluconic acid, and then water was added to give a total volume of 100 mL, thus obtaining a disinfectant and/or bactericidal aqueous composition.

### Control Example 2

Water was added to 2 ml of a 10 W/V% benzalkonium chloride aqueous solution (Nihon Pharmaceutical Co., Ltd.) to give a total volume of 100 mL, thus obtaining a disinfectant and/or bactericidal aqueous composition.

### Control Example 3

Water was added to 5 ml of a 20 W/V% chlorhexidine gluconate aqueous solution (Sumitomo Pharmaceuticals Company Limited) to give a total volume of 100 mL, thus obtaining a disinfectant and/or bactericidal aqueous composition.

### [Evaluation methods]

### <Safety tests>

For each of the disinfectant and/or bactericidal aqueous compositions obtained in the Examples and Control Examples described above, a local skin safety test was carried out as follows in accordance with the Draize method using rabbits (male NZW).

The hair on the back of each of the rabbits was shorn off, and rabbits having little island skin and for which the hair cycle was in the dormant period were selected, and six rabbits were put into each group. The back of each rabbit was divided into four zones, and two zones with point symmetry relative to the center were taken as unwounded skin, and the other two zones were taken as wounded skin by forming 2.5 cm-long scratches in a double cross shape using an 18G injection needle on the day of application. A 2.5 cm-square lint cloth onto which 0.3 ml of the test liquid had been applied was stuck onto the site of application using rayon tape, and fixing was carried out by covering over with taping tape. 24 hours after the application, the lint cloth was removed.

The site of application was examined, and an evaluation score was determined for erythema, incrustation and edema in accordance with the Draize judgement criteria. The primary irritation index (P.I.I) was calculated from the evaluation scores for the unwounded skin and the wounded skin 24 hours and 72 hours after the application, thus evaluating the irritability. The results are shown in Table 1.

**Table 1**

| Composition | P.I.I |
|---|---|
| Example 1 | 0.7 |
| Example 2 | 0.4 |
| Example 3 | 0.3 |
| Example 4 | 0.4 |
| Example 5 | 0.3 |
| Control Example 1 | 0.6 |

The skin irritability was judged from the index (P.I.I) using the following criteria.
0 ≤ P.I.I. ≤ 2: Weak irritation
2 < P.I.I. ≤ 5: Medium irritation
5 < P.I.I. ≤ 8: Strong irritation

The results show that the compositions of Examples 1 to 5 according to the present invention have approximately the same or lower skin irritation than the compositions of Control Example 1, which are already widely used as disinfectants, and thus are highly safe.

In addition, the compositions of Examples 1 to 5 also have the same or lower skin irritation than the composition of Control Examples 2 to 3.

### <Antibacterial activity tests>

Each test strain was cultured overnight at 37°C using a Muller-Hinton broth, successive subculture was carried out three times, and then the resulting precultured bacterial solution was adjusted to an optical density (OD) at 660nm of 0.3 Abs using sterilized distilled water to make the count approximately 10⁸ cfu/mL, and then the solution was further diluted to 100 fold with sterilized distilled water to make the count approximately 10⁶ cfu/mL, thus obtaining a test bacterial suspension.

Using the test suspension, a 2-fold dilution series was produced using sterilized distilled water such that for each suspension in the series the olanexidine concentration was 2 times of the final test concentration, and 50 µL of each suspension in the series was dispensed into the wells of eight rows in length of a 96-well microplate in order of lowest concentration upward. 50 µL of the test bacterial suspension was dispensed into each of the wells into which one of the suspension to be tested had been dispensed, and mixing was carried out immediately. 10 µL of each mixed reaction liquid was collected, and after a prescribed treatment time period was instilled into 200 µL of an SCDLP culture medium (deactivating medium for disinfectant) that had already been dispensed into the wells of another 96-well microplate and mixing was carried out to stop the bactericidal activity, and then culturing was carried out for 48 hours at 37°C.

After the culturing, it was judged with the naked eye from turbidity of the culture medium whether or not the bacteria had proliferated in each well, with the presence of turbidity being taken as indicating that the bacteria had proliferated, and the absence of turbidity being taken as indicating that the bacteria had not proliferated. Out of the dilution series for the test suspension, the minimum concentration for which proliferation of the bacteria was not observed was taken as the minimum bactericidal concentration (MBC) of the liquid to be tested for the test bacteria.

The antibacterial activity test results are shown in Tables 2 and 3.

**Table 2**

| MBC for 10-second treatment (µg/mL) | | | | | | |
|---|---|---|---|---|---|---|
| Test strain | Example 1 | Example 2 | Example 3 | Example 4 | Example 5 | Control Example 1 |
| *Staphylococcus aureus* | ≤ 7.81 | 31.3 | 62.5 | 62.5 | 31.3 | 5000 |
| FDA 209P | | | | | | |
| MRSA | ≤ 7.81 | 31.3 | 31.3 | 15.6 | 62.5 | > 5000 |
| Clinical isolates C5414 | | | | | | |
| *Staphylococcus epidermidis* | ≤ 7.81 | ≤ 7.81 | ≤ 7.81 | ≤ 7.81 | 15.6 | 500 |
| ATCC 12228 | | | | | | |
| *Enterococcus faecalis* | ≤ 7.81 | 15.6 | ≤ 7.81 | 15.6 | 15.6 | > 5000 |
| NCTC 12201 (VRE) | | | | | | |
| *Escherichia coli* | ≤ 7.81 | 15.6 | 15.6 | 15.6 | 15.6 | 2000 |
| NIHJ JC-2 | | | | | | |
| *Serratia marcescens* | ≤ 7.81 | ≤ 7.81 | ≤ 7.81 | ≤ 7.81 | ≤ 7.81 | 5000 |
| IFO 12648 | | | | | | |
| *Proteus mirabilis* | ≤ 7.81 | 250 | > 500 | > 500 | > 500 | > 5000 |
| 1287 | | | | | | |
| *Burkholderia cepacia* | ≤ 7.81 | > 500 | > 500 | > 500 | > 500 | > 5000 |
| IFO 14595 | | | | | | |

**Table 3**

| MBC for 180-second treatment (µg/mL) | | | | | | |
|---|---|---|---|---|---|---|
| Test strain | Example 1 | Example 2 | Example 3 | Example 4 | Example 5 | Control Example 1 |
| *Staphylococcus aureus* | ≤ 7.81 | ≤ 7.81 | ≤ 7.81 | ≤ 7.81 | ≤ 7.81 | 2000 |
| FDA 209P | | | | | | |
| MRSA | ≤ 7.81 | < 7.81 | ≤ 7.81 | ≤ 7.81 | ≤ 7.81 | > 5000 |
| Clinical isolates C5414 | | | | | | |
| *Staphylococcus epidermidis* | ≤ 7.81 | ≤ 7.81 | ≤ 7.81 | ≤ 7.81 | ≤ 7.81 | < 7.81 |
| ATCC 12228 | | | | | | |
| *Enterococcus faecalis* | ≤ 7.81 | ≤ 7.81 | ≤ 7.81 | ≤ 7.81 | ≤ 7.81 | > 5000 |
| NCTC 12201 (VRE) | | | | | | |
| *Escherichia coli* | ≤ 7.81 | ≤ 7.81 | ≤ 7.81 | ≤ 7.81 | ≤ 7.81 | 31.3 |
| NIHJ JC-2 | | | | | | |
| *Serratia marcescens* | ≤ 7.81 | ≤ 7.81 | ≤ 7.81 | ≤ 7.81 | ≤ 7.81 | ≤ 7.81 |
| IFO 12648 | | | | | | |
| *Proteus mirabilis* | ≤ 7.81 | 15.6 | ≤ 7.81 | ≤ 7.81 | ≤ 7.81 | 1000 |
| 1287 | | | | | | |
| *Burkholderia cepacia* | ≤ 7.81 | 125 | 31.3 | 62.5 | 62.5 | 5000 |
| IFO 14595 | | | | | | |

As shown by the results in Tables 2 and 3, the compositions of Examples 1 to 5 according to the present invention show a broad antibacterial spectrum, and show stronger bactericidal effects than the conventional disinfectant. Moreover, as shown by the results in Table 2, these bactericidal effects are shown strongly even with a short bactericidal treatment time of 10 seconds, which is very advantageous in terms of practical use.

The solubility of olanexidine hydrochloride alone is 0.02% at 0°C, and, for example, at this concentration, the bactericidal activity is prone to being influenced by pH, serum, soap, temperature and so on. With the compositions of the present invention, the olanexidine hydrochloride concentration is increased, and hence such influence is eliminated.

### Test Example 1

For the composition of Example 2 [concentration 0.1% (1000 µg/mL) in terms of olanexidine hydrochloride], and an aqueous solution of 0.02% (200 µg/mL) olanexidine hydrochloride alone, a comparison of the bactericidal activity under conditions of contamination with organic matter (under the presence of 5% bovine serum) was carried out. *Staphylococcus* aureus FDA 209P, *Escherichia coli* NIHJ JC-2 and *Pseudomonas aeruginosa* ATCC 10145 were used as test bacteria. The results are shown in Table 4.

**Table 4**

| | Bovine serum concentration (%) | Aqueous solution of olanexidine hydrochloride alone | | | Example 2 (0.32% NP-10) | | |
|---|---|---|---|---|---|---|---|
| | | 30 s | 1 min | 3 min | 30 s | 1 min | 3 min |
| *Staphylococcus* aureus | 0 | 25 | 6.25 | ≤ 3.13 | 25 | 12.5 | 12.5 |
| FDA 209P | 5 | > 200 | 100 | 100 | 1000 | 100 | 100 |
| *Escherichia coli* | 0 | 6.25 | 3.13 | ≤ 3.13 | 15.6 | 7.81 | 7.81 |
| NIHJ JC-2 | 5 | > 200 | > 200 | > 200 | 1000 | 1000 | 500 |
| *Pseudomonas aeruginosa* | 0 | 6.25 | ≤ 3.13 | ≤ 3.13 | 6.25 | ≤3.13 | ≤ 3.13 |
| ATCC 10145 | 5 | > 200 | > 200 | > 200 | 1000 | 1000 | 1000 |

From these results, it was found that even under the presence of organic matter, the composition of Example 2 exhibits bactericidal activity in a shorter time than the aqueous solution of olanexidine hydrochloride alone, and hence is highly practically useful.

### Test Example 2

The skin on the back of mice was contaminated with *Staphylococcus aureus* FDA 209P, and a comparison of the disinfectant effect was carried out for various treatment groups as shown in Table 5.

**Table 5**

| Treatment group (No. n = 6) | 10 s after (immediately after) disinfecting | | 2 hr after disinfecting | |
|---|---|---|---|---|
| | No. bacteria survived | Eradication rate (%) | No. bacteria survived | Eradication rate |
| Untreated group | 8.45 x 10⁻³ | 0 | 9.06 x 10³ | 0 |
| Group treated with 70% ethanol | 0 | 100 | 8.99 x 10³ | 0.77 |
| Group treated with composition of 1 | 0 | 100 | 4 | 99.9 |
| Group treated with composition of 2 | 8 | 99.9 | 3 | 99.9 |

The results show that immediately after (10 s after) the disinfecting, the group treated with 70% ethanol and the group treated with the composition of Example 1 exhibited a eradication rate of 100%, and the group treated with the composition of Example 2 exhibited a sterilization rate of almost 100%. However, two hours after the disinfecting, the group treated with 70% ethanol exhibited a sterilization rate of 0.77%, which was close to that for the untreated group, i.e. a sterilization effect was no longer observed. In contrast with this, the groups treated with the compositions of Examples 1 and 2 each exhibited a sterilization rate of at least 99.9% two hours after the disinfecting, i.e. exhibited a high sterilization sustaining effect. This sterilization sustaining effect was still observed even four hours after the disinfecting.

### Compositions of invention III

### Example III-1

Approximately 80 ml of water was added to 1 g of an olanexidine hydrochloride powder (Otsuka Pharmaceutical Co., Ltd.) and 3.1 g of polyoxyethylene (POE (20)) sorbitan monooleate (made by Nikko Chemicals Co., Ltd.) and mixing and dissolution were carried out, the pH was adjusted to 5, and then water was further added to give a total volume of 100 mL, thus obtaining a disinfectant and/or bactericidal aqueous composition.

### Example III-2

Approximately 80 ml of water was added to 1 g of an olanexidine hydrochloride powder (Otsuka Pharmaceutical Co., Ltd.) and 7.4 g of a decaglyceryl monolaurate (made by Nikko Chemicals Co., Ltd.) and mixing and dissolution were carried out, the pH was adjusted to 5, and then water was further added to give a total volume of 100 mL, thus obtaining a disinfectant and/or bactericidal aqueous composition.

### Example III-3

Approximately 80 ml of water was added to 1 g of an olanexidine hydrochloride powder (Otsuka Pharmaceutical Co., Ltd.) and 4.9 g of a polyoxyethylene (POE (7)) glyceryl cocoate (made by Cognis Japan Ltd.) and mixing and dissolution were carried out, the pH was adjusted to 5, and then water was further added to give a total volume of 100 mL, thus obtaining a disinfectant and/or bactericidal aqueous composition.

### Example III-4

Approximately 80 ml of water was added to 1 g of an olanexidine hydrochloride powder (Otsuka Pharmaceutical Co., Ltd.) and 4.8 g of a polyoxyethylene (POE (7)) glyceryl caprylate/caprate (made by Cognis Japan Ltd.) and mixing and dissolution were carried out, the pH was adjusted to 5, and then water was further added to give a total volume of 100 mL, thus obtaining a disinfectant and/or bactericidal aqueous composition.

### Example III-5

Approximately 80 ml of water was added to 1 g of an olanexidine hydrochloride powder (Otsuka Pharmaceutical Co., Ltd.) and 11.1 g of a polyoxyethylene (POE (120)) methyl glucoside dioleate (made by Amerchol) and mixing and dissolution were carried out, the pH was adjusted to 5, and then water was further added to give a total volume of 100 mL, thus obtaining a disinfectant and/or bactericidal aqueous composition.

### Example III-6

Approximately 80 ml of water was added to 1 g of an olanexidine hydrochloride powder (Otsuka Pharmaceutical Co., Ltd.) and 9.1 g of α-cyclodextrin (made by Hayashibara) and mixing and dissolution were carried out, the pH was adjusted to 5, and then water was further added to give a total volume of 100 mL, thus obtaining a disinfectant and/or bactericidal aqueous composition.

The aqueous solutions obtained in Examples III-1 to III-6 were still transparent and colorless without precipitation even after being left at a temperature in the vicinity of 0°C.

### Comparative Example III-1

Approximately 80 ml of water was added to 50 mg of olanexidine hydrochloride (Otsuka Pharmaceutical Co., Ltd.) and dissolution was carried out, the pH was adjusted to 6, and then water was further added to give a total volume of 100 mL, thus obtaining a disinfectant and/or bactericidal aqueous composition. (Solubility was 0.05 W/V% at 25°C.)

### Control III-1

Approximately 80 ml of water was added to 2.5 ml of a 20 W/V% chlorhexidine gluconate aqueous solution (Sumitomo Pharmaceuticals Company Limited), the pH was adjusted to 6, and then water was further added to give a total volume of 100 mL, thus obtaining a disinfectant and/or bactericidal aqueous composition.

### <Antibacterial activity tests>

Each test strain was cultured overnight at 37°C using a Muller-Hinton broth, successive subculture was carried out three times, and then the resulting precultured bacterial solution was adjusted to an optical density (OD) at 660nm of 0.3 Abs using sterilized distilled water to make the count approximately 10⁸ cfu/mL, and then the solution was further diluted to 100 fold with sterilized distilled water to make the count approximately 10⁶ cfu/mL, thus obtaining a test bacterial solution.

Using the test suspension, a 2-fold dilution series was produced using sterilized distilled water such that for each suspension in the series the olanexidine concentration was 2 times of the final test concentration, and 50 µL of each liquid in the series was dispensed into the wells of eight rows in length of a 96-well microplate in order of lowest concentration upward. 50 µL of the test bacterial solution was dispensed into each of the wells into which one of the test liquids had been dispensed, and mixing was carried out immediately. 10 µL of each mixed reaction suspension was collected, and after a prescribed treatment time period was instilled into 200 µL of an SCDLP culture medium (deactivating medium for disinfectant) that had already been dispensed into the wells of another 96-well microplate and mixing was carried out to stop the bactericidal activity, and then culturing was carried out for 48 hours at 37°C.

After the culturing, it was judged with the naked eye from turbidity of the culture medium whether or not the bacteria had proliferated in each well, with the presence of turbidity being taken as indicating that the bacteria had proliferated, and the absence of turbidity being taken as indicating that the bacteria had not proliferated. Out of the dilution series for the test suspension, the minimum concentration for which proliferation of the bacteria was not observed was taken as the minimum bactericidal concentration (MBC) of the test liquid for the test bacteria.

The antibacterial activity test results are shown in Tables 6 and 7.

**Table 6 (MBC (µg/mL)**

| Test strain (exp: 10⁶CFU/mL) | Staphylococcus aureus | | Staphylococcus epidermidis | | Enterococcus faecalis | |
|---|---|---|---|---|---|---|
| | FDA 209P | | ATCC 12228 | | NCTC 12201 | |
| Treatment time | 30 s | 3 min | 30 s | 3 min | 30 s | 3 min |
| Comparative Example III-1 | 62.5 | 15.63 | 7.81 | 7.81 | 15.63 | 7.81 |
| Example III-1 | 500 | 25 | ≤ 7.81 | ≤ 7.81 | ≤ 7.82 | ≤ 7.81 |
| Example III-2 | 62.5 | ≤ 7.81 | ≤ 7.81 | ≤ 7.81 | < 7.81 | ≤ 7.81 |
| Example III-3 | 5000 | 2000 | 250 | ≤ 7.81 | 62.5 | 31.25 |
| Example III-4 | 31.25 | ≤ 7.81 | ≤ 7.81 | ≤ 7.81 | ≤ 7.82 | ≤ 7.81 |
| Example III-5 | 5000 | 2000 | 250 | ≤ 7.81 | 62.5 | 31.25 |
| Example III-6 | 62.5 | ≤ 7.81 | ≤ 7.81 | ≤ 7.81 | 15.63 | ≤ 7.81 |
| Control Example III-1 | 5000 | 2000 | 500 | ≤ 7.81 | > 5000 | > 5000 |

**Table 7 (MBC (µg/mL)**

| Test strain (exp: 10⁶CFU/mL) | Escherichia coli | | Serratia marcescens | |
|---|---|---|---|---|
| | NIHJ JC-2 | | IFO 12648 | |
| Treatment time | 30 s | 3 min | 30 s | 3 min |
| Comparative Example III-1 | 15.63 | 7.81 | 15.63 | 7.81 |
| Example III-1 | 12.5 | ≤ 7.81 | 12.5 | ≤ 7.81 |
| Example III-2 | 15.63 | ≤ 7.81 | ≤ 7.81 | ≤ 7.81 |
| Example III-3 | 125 | 31.25 | 500 | ≤ 7.81 |
| Example III-4 | ≤ 7.81 | ≤ 7.81 | ≤ 7.81 | ≤ 7.81 |
| III-5 | 125 | 31.25 | 500 | ≤ 7.81 |
| Example III-6 | 15.63 | 15.63 | ≤ 7.81 | ≤ 7.81 |
| Control Example III-1 | 500 | 31.25 | 1000 | ≤ 7.81 |

As shown by the results in Tables 6 and 7, the liquids of Examples III-1 to III-6 according to the present invention show a broad antibacterial spectrum than the liquid of Comparative Example III-1, and show stronger bactericidal effects than the conventional disinfectant (Control Example III-1). Moreover, these bactericidal effects are displayed strongly even with a relatively short bactericidal treatment time of 30 seconds, which is very advantageous in terms of practical use.

The solubility in water of olanexidine hydrochloride alone is 0.02% at 0°C, and, for example, at this concentration, the bactericidal activity is prone to being influenced by pH, serum, soap, temperature and so on. With the compositions of the present invention, the olanexidine hydrochloride concentration is increased, and hence such influence is eliminated.

## Claims

1. A disinfectant and/or bactericidal aqueous composition containing an olanexidine acid addition salt and a cyclodextrin.

2. The disinfectant and/or bactericidal aqueous composition according to claim 1, wherein the cyclodextrin is selected from the group consisting of α-cyclodextrin, β-cyclodextrin and γ-cyclodextrin.

3. The disinfectant and/or bactericidal aqueous composition according to claim 1, wherein the olanexidine acid addition salt is present at a concentration of 0.05 to 1 W/V% and the cyclodextrin is present at a concentration of 0.1 to 10 W/V%.

4. The disinfectant and/or bactericidal aqueous composition according to claim 1, wherein the olanexidine acid addition salt is present at a concentration of 0.1 to 1 W/V% and the cyclodextrin is present at a concentration of 0.1 to 10 W/V%.

5. The disinfectant and/or bactericidal aqueous composition according to claim 1, wherein the composition further contains an alcohol.

6. The disinfectant and/or bactericidal aqueous composition according to claim 5, wherein the alcohol is present in the composition at a concentration of 20 to 80 W/V %.

7. A method of sterilizing or disinfecting an object, comprising contacting the object with an effective amount of the disinfectant and/or bactericidal aqueous composition according to claim 1.

8. Use of a disinfectant and/or bactericidal aqueous composition according to claim 1 for sterilization or disinfection.
